# EUROPEAN PATENT APPLICATION

(11) **EP 4 428 242 A1**
(43) Date of publication of application: **11.09.2024**
(21) Application number: 22889974.6
(22) Date of filing: 02.11.2022
(51) Int. Cl.: C12Q 1/02, C12Q 1/6851, C12Q 1/686, G01N 33/50

(54) **METHOD FOR DETERMINING RISK OF ADVERSE EVENT**

(30) Priority: 05.11.2021 JP 2021181204
(71) Applicant: Kabushiki Kaisha Yakult Honsha, Tokyo, 105-8660 (JP)
(72) Inventor: ASAHARA, Takashi, Tokyo 105-8660 (JP); SUGIMOTO, Takuya, Tokyo 105-8660 (JP)
(74) Representative: Wächtershäuser & Hartz Patentanwaltspartnerschaft mbB
(86) International application number: PCT/JP2022/040978
(87) International publication number: WO 2023/080154

(57) **Abstract**

A method for determining a risk of an adverse event during multimodal treatment is provided. A method for determining a risk of an adverse event during multimodal treatment includes measuring *Anaerostipes hadrus* in a sample collected from a subject.

## Description

### Technical Field

The present invention relates to a method for determining a risk of an adverse event during multimodal treatment.

### Background Art

In a treatment of progressive esophageal cancer, a combination of preoperative chemotherapy and surgery has been one of promising treatment strategies, but in preoperative chemotherapy, serious adverse events such as diarrhea and febrile neutropenia occur. An onset of such adverse events leads to a discontinuation or reduction of an anticancer drug, making it difficult to obtain a sufficient antitumor effect, and in addition, causes deterioration of an immunity and deterioration of a nutritional state, leading to deterioration of quality of life (QOL) of a patient. Therefore, reduction of adverse events in chemotherapy is an important issue.

Hitherto, it has been reported that the onset of febrile neutropenia and severe diarrhea are significantly reduced by allowing a patient to ingest synbiotics during performing a DCF therapy (a three-drug combination therapy of docetaxel, cisplatin, and 5-fluorouracil) as a preoperative chemotherapy for esophageal cancer (Non Patent Literature 1). Meanwhile, if occurrence of adverse events can be predicted before treatment, preventive measures such as examination of amount of an anticancer agent to be used and combined use of synbiotics can be considered in advance.

*Anaerostipes hadrus* is one of dominant bacteria in an intestine, and is known as a bacterium that produces butyric acid using lactic acid and acetic acid in the intestine. In addition, it has been reported that in a healthy subject, deletion of an inositol catabolism and butyric acid biosynthesis pathway of *A*. *hadrus* is associated with an increase in host weight and risk of metabolic disease (Non Patent Literature 2). However, there is no known association between *A*. *hadrus* and adverse events upon treatment such as chemotherapy.

### Citation List

### Non Patent Literature

Non Patent Literature 1: Motoori M, et al. Clin Nutr, 2017, 36:93-99
Non Patent Literature 2: Zeevi D, et al. Nature, 2019, 568:43-48

### Summary of Invention

### Technical Problem

In order to effectively treat a disease, it is desirable to determine a risk of onset or exacerbation of an adverse event during treatment to start treatment. Therefore, an object of the present invention is to provide a method for determining the risk of an adverse event during multimodal treatment.

### Solution to Problem

As a result of intensive studies focusing on intestinal bacteria, the present inventors have found that there is a correlation between *Anaerostipes hadrus*, which is a type of intestinal bacteria, and a presence or absence of the onset or severity of the adverse event during the treatment, and it is possible to determine the risk of an adverse event during the multimodal treatment using *Anaerostipes hadrus* as an index.

In other words, the present invention provides (1) to (12) below.
(1) A method for determining a risk of an adverse event during multimodal treatment, the method including measuring *Anaerostipes hadrus* in a sample collected from a subject.
(2) The method according to (1), wherein the risk of an adverse event is a risk of onset or exacerbation of an adverse event.
(3) The method according to (1) or (2), wherein the multimodal treatment is a treatment selected from a drug therapy, a surgical therapy, radiation treatment, and a combination thereof.
(4) The method according to any one of (1) to (3), wherein the multimodal treatment is a drug therapy.
(5) The method according to any one of (1) to (4), wherein the adverse event is at least one selected from the group consisting of febrile neutropenia and diarrhea.
(6) The method according to any one of (1) to (5), wherein the sample is a fecal sample of the subject.
(7) The method according to any one of (1) to (6), further including comparing the number of bacteria of *Anaerostipes hadrus* in the sample with a reference value, and determining that the risk of an adverse event during the multimodal treatment is high when the number of bacteria is equal to or less than the reference value.
(8) The method according to any one of (1) to (6), further including comparing an occupancy rate of *Anaerostipes hadrus* in total bacteria in the sample with a reference value, and determining that the risk of an adverse event during the multimodal treatment is high when the occupancy rate is equal to or less than the reference value.
(9) The method according to any one of (1) to (6), further including determining that a risk of febrile neutropenia during the multimodal treatment is high when the number of bacteria of *Anaerostipes hadrus* in the sample is 10^{7.7} or less per gram of the sample or an occupancy rate of *Anaerostipes hadrus* in total bacteria in the sample is 0.25% or less.
(10) The method according to any one of (1) to (6), further including determining that a risk of diarrhea during the multimodal treatment is high when the number of bacteria of *Anaerostipes hadrus* in the sample is 10^{7.6} or less per gram of the sample or an occupancy rate of *Anaerostipes hadrus* in total bacteria in the sample is 0.084% or less.
(11) A kit for performing the method according to any one of (1) to (10), including a measurement reagent and a protocol for *Anaerostipes hadrus* in a sample.
(12) A method for screening for an agent for reducing a risk of an adverse event during multimodal treatment, the method including using the number of bacteria or occupancy rate of *Anaerostipes hadrus* as an index.

### Advantageous Effects of Invention

According to the present invention, it is possible to easily determine the risk of onset or exacerbation of an adverse event during the multimodal treatment. This makes it possible to take measures against the adverse events, such as selection of treatment to be performed, adjustment of drug amount, and prevention of adverse events, depending on a level of risk, and to select an appropriate treatment regimen for each individual patient.

### Brief Description of Drawings

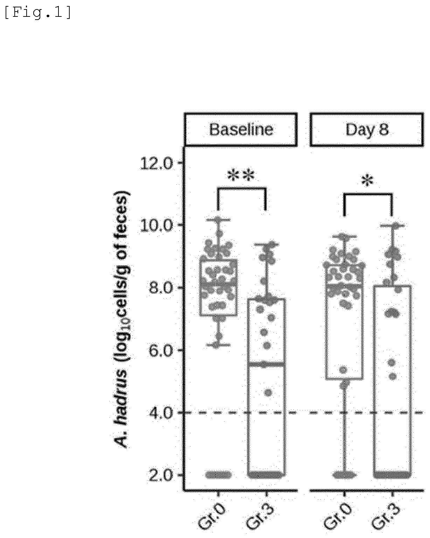

Fig. 1 is a graph showing a comparison of the number of bacteria of *A*. *hadrus* depending on a presence or absence of onset of febrile neutropenia. Gr. 0 (grade 0) indicates patients who did not develop febrile neutropenia and Gr.3 (grade 3) indicates patients who developed febrile neutropenia. Baseline indicates before the DCF therapy, and Day 8 indicates 8th day of one course of the DCF therapy. *p<0.05, **p<0.01.

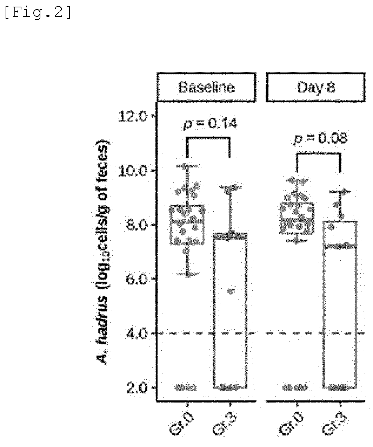

Fig. 2 is a graph showing a comparison of the number of bacteria of *A*. *hadrus* depending on the presence or absence of onset of febrile neutropenia in a synbiotics administration group. Gr. 0 (grade 0) indicates patients who did not develop febrile neutropenia and Gr.3 (grade 3) indicates patients who developed febrile neutropenia. Baseline indicates before the DCF therapy, and Day 8 indicates 8th day of one course of the DCF therapy.

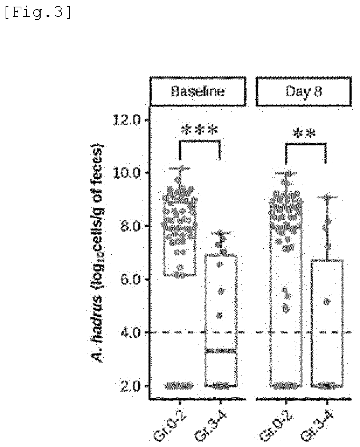

Fig. 3 is a graph showing a comparison of the number of bacteria of *A*. *hadrus* according to the severity of diarrhea. Gr.0-2 (grade 0 to 2) refers to patients with relatively low severity of diarrhea and Gr.3-4 (grade 3 to 4) refers to patients with very high severity of diarrhea. Baseline indicates before the DCF therapy, and Day 8 indicates 8th day of one course of the DCF therapy. **p<0.01, ***p<0.001.

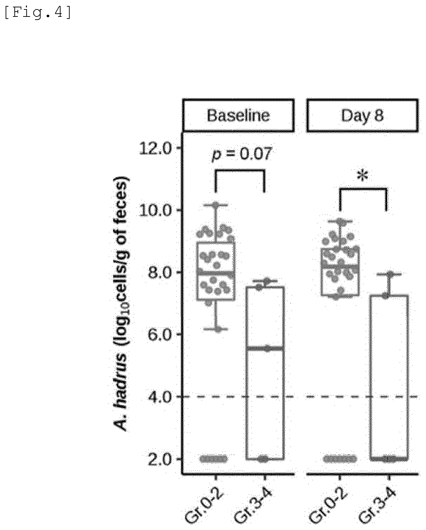

Fig. 4 is a graph showing a comparison of the number of bacteria of *A*. *hadrus* depending on the severity of diarrhea in the synbiotics administration group. Gr.0-2 (grade 0 to 2) refers to patients with relatively low severity of diarrhea and Gr.3-4 (grade 3 to 4) refers to patients with very high severity of diarrhea. Baseline indicates before the DCF therapy, and Day 8 indicates 8th day of one course of the DCF therapy. *p<0.05.

### Description of Embodiments

"*Anaerostipes hadrus* (*Eubacterium hadrum*)" is a Gram-positive bacterium and is a type of intestinal bacteria in humans. As used in the present description, "*Anaerostipes hadrus*" means a bacterium belonging to *Anaerostipes hadrus.*

In the description, the term "multimodal treatment" includes a drug therapy using a drug such as an anticancer agent, a surgical therapy such as surgery, a radiation treatment, and a combination thereof.

As used in the description, "during the treatment" includes during and after the treatment.

As used in the description, the term "adverse event" refers to an unfavorable or unintended sign, symptom or disease that occurs in a patient during or after the treatment whether or not there is a causal relationship with the treatment. Examples of adverse events and their severity include adverse events and their grade in Common Terminology Criteria for Adverse Events (CTCAE) v4.0 published by National Cancer Institute (NCI).

The severity of adverse events is classified, according to CTCAE v4.0, into the following grades 1 to 5 or a part thereof.

Grade 1: mild; no symptoms or mild symptoms; only clinical or laboratory findings; no treatment is required.

Grade 2: moderate; minimal/local/non-invasive treatment is required; limitation of activities of daily living other than age-appropriate self care activity.

Grade 3: severe or medically significant but not immediately life threatening; hospitalization or prolongation of period of hospitalization is required; inactive or inoperable; limitation of self care activities of daily living.

Grade 4: life threatening; emergency treatment is required.

Grade 5: death due to adverse events.

Incidentally, in the description, no onset is defined as grade 0.

As used in the description, the "risk of an adverse event" refers to the risk of onset or exacerbation of an adverse event.

"Risk of onset of an adverse event" refers to a likelihood of developing an adverse event. In other words, a fact that an individual belongs to a high-risk group means that the individual is expected to have a high possibility of developing an adverse event, and a fact that an individual belongs to a low-risk group means that the individual is expected to have a low possibility of developing an adverse event.

In addition, the "risk of exacerbation of an adverse event" refers to a possibility of exacerbation of an adverse event. In other words, an individual belonging to the high-risk group means that the individual is expected to have a high possibility of onset of an adverse event and a possibility of exacerbation of the adverse event is expected to be high, and an individual belonging to the low-risk group means that the individual is expected to have a low possibility of developing an adverse event, or even if the individual has developed an adverse event, the individual is expected to have a low possibility of exacerbation of the adverse event.

In the present description, "determination" includes a concept of "detection", "examination", "measurement", "prediction", or "diagnosis", but does not include medical practice such as diagnosis by a doctor.

In the method for determining the risk of an adverse event during the multimodal treatment according to the present invention, *Anaerostipes hadrus* in a sample is used as an index. The method includes measuring *A*. *hadrus* in the sample. Specifically, the method includes measuring the number of bacteria of *A*. *hadrus* in a sample collected from a subject. Alternatively, the method includes measuring an occupancy rate of *A*. *hadrus* in total bacteria in the sample collected from the subject. Here, the occupancy rate of *A*. *hadrus* in total bacteria in the sample means a ratio of the number of bacteria of *A*. *hadrus* to the total number of bacteria in the sample, and is a value corresponding to the occupancy rate of *A*. *hadrus* in the intestinal bacterial flora of the subject from which the sample is derived.

The subject is not particularly limited, and examples thereof include those requiring determination of the risk of an adverse event during the multimodal treatment, such as a patient who is scheduled to receive the multimodal treatment and a patient who is receiving the multimodal treatment. Examples of the sample include biological samples derived from a subject, for example, digestive tract contents such as intestinal fluid and feces, and feces are preferable in that a burden on the subject is small.

A means for measuring the number of bacteria of *A*. *hadrus* in the sample is not particularly limited, but preferably includes a means for measuring by, for example, an RT-PCR method or a sequencing method based on a base sequence of the 16S rRNA gene of *A*. *hadrus,* and among them, it is more preferable to measure by the RT-PCR method.

Here, the RT-PCR method will be described. An analysis method using the RT-PCR method can be performed by, for example, (1) a step of extracting RNA of intestinal bacteria in a sample, (2) a step of synthesizing cDNA by reverse transcription from the extracted RNA and performing PCR using a nucleic acid fragment (a primer) that hybridizes with cDNA derived from *A*. *hadrus,* and (3) a step of detecting a DNA fragment amplified in step (2). By combining the nucleic acid fragment with a template cDNA derived from the sample and performing an amplification reaction, the DNA fragment (a PCR product) specific to *A*. *hadrus* can be obtained. By observing the PCR product over time and specifying the number of PCR cycles when a certain amount of DNA is reached, the number of bacteria of *A*. *hadrus* in the sample can be quantified.

A temporal observation of the PCR product amplified can be performed by labeling the PCR product with an intercalating fluorescent dye such as SYBR (registered trademark) Green I and measuring a fluorescence intensity at each PCR stage. Since an intercalating dye has a property of increasing the fluorescence intensity by intercalating into a double-stranded nucleic acid, it is possible to accurately measure the PCR product generated by a PCR reaction from cDNA of *A*. *hadrus,* and in particular, SYBR Green I is suitably used.

By specifying the number of PCR cycles (hereinafter, referred to as a Cq value) when a predetermined certain fluorescence intensity (the DNA amount) is reached, it is possible to quantify *A*. *hadrus* in a sample. In addition, for example, a TaqMan (registered trademark) probe or a Moleculer Beacon labeled with a fluorescent dye can also be used. A TaqMan probe and the Moleculer Beacon are probes in which a fluorescent dye and a quencher are bound to an oligonucleotide having homology with an internal sequence of a region amplified by PCR, and are used in a state of coexisting in a PCR reaction. Since the fluorescence corresponding to a PCR amplification reaction is emitted by an interaction between the fluorescent dye and the quencher which are bound to the probe, it is possible to observe the amplified PCR product over time by measuring the fluorescence intensity at each PCR stage.

The number of bacteria of *A*. *hadrus* in the sample can be determined from a logarithmic value of the number of bacteria measured by, for example, a DAPI count method or a culture method and a calibration curve of the Cq value. In other words, the calibration curve in which the logarithmic value of the number of bacteria of *A*. *hadrus* is plotted on a horizontal axis and the Cq value is plotted on a vertical axis is prepared in advance, and the Cq value obtained as a result of the PCR reaction is applied to the calibration curve to measure the number of bacteria of *A*. *hadrus* in the sample.

In the measurement of the number of bacteria of *A*. *hadrus* in the sample, in the PCR reaction, a primer that can specifically hybridize with and amplify a region that is preserved in the species of *A*. *hadrus* on the 16S rRNA gene of *A*. *hadrus* and is not preserved in species other than *A*. *hadrus* may be used. The primers for measuring the number of bacteria of *A*. *hadrus* are not limited thereto, and for example, primers of SEQ ID NOs: 3 and 4 can be used.

The means for measuring the occupancy rate of *A*. *hadrus* in total bacteria in the sample is not particularly limited, but preferably includes the means for measuring by, for example, the RT-PCR method or the sequencing method based on the base sequence of the 16S rRNA gene of intestinal bacteria, and among them, it is more preferable to measure by the sequencing method (for example, a 16S rRNA gene amplicon analysis).

Here, the 16S rRNA gene amplicon analysis will be described. The 16S rRNA gene amplicon analysis can be performed by, for example, (1) a step of extracting genomic DNA of intestinal bacteria in a sample, (2) a step of performing PCR using the extracted genomic DNA as a template and using the nucleic acid fragment (the primer) that hybridizes with the 16S rRNA gene of intestinal bacteria, (3) a step of determining the base sequence of the DNA fragment amplified in the step (2), and (4) a step of analyzing sequence data obtained in the step (3). The DNA fragment (the PCR product) derived from the 16S rRNA gene of intestinal bacteria can be obtained by combining the nucleic acid fragment with a template genomic DNA derived from the sample and performing the amplification reaction. Furthermore, after sequencing of the PCR product and removal of the error sequence, the sequence data are summarized as amplicon sequence variants (ASV) and phylogenetic information is assigned to each ASV by making reference to a known database, and whereby a type and abundance ratio of intestinal bacteria contained in the sample can be analyzed. Based on the information obtained, it is possible to calculate the occupancy rate of *A*. *hadrus* with respect to total intestinal bacteria.

A region of the 16S rRNA gene of intestinal bacteria amplified by the PCR is preferably a region which is amplified using a primer that hybridizes with a conserved region that is universally conserved between bacterial species, and which includes a variable region rich in change that is not conserved between bacterial species in the region. The variable region includes at least one region of the V1 to V9 regions of the 16s rRNA gene, and is preferably a region including V1 and V2 or a region including V3 and V4. The primer may optionally contain an adaptor sequence for sequencing and/or an index sequence for sample identification. Examples of such a primer include a universal primer for amplification of a bacterial 16S rRNA gene usually used in the art, and for example, primers of SEQ ID NOs: 1 and 2 can be used.

The base sequence of the amplified PCR product can be determined by a known method, but can be quickly sequenced by using a next-generation sequencer, for example, MiSeq platform (Illumina). Analysis of the sequence data can be performed using analysis software such as QIIME2 (Quantitative Insights Into Microbial Ecology 2), and a sequence error can be removed using, for example, a DADA2 (Divisive Amplicon Denoising Algorithm 2) plug-in of QIIME2. The obtained sequence data can be classified into ASVs based on sequence identity, and assignment to a bacterial species of each ASV can be determined with reference to a known database such as SILVA or Greengenes. From the bacterial species of each ASV determined in this way and their presence ratio corresponding to the number of sequence reads, the occupancy rate of *A*. *hadrus* with respect to the total intestinal bacteria can be calculated.

As shown in the examples to be described later, a significant negative correlation was observed between the occupancy rate of *A*. *hadrus* in the intestinal bacterial flora after administration of the three-drug combination therapy (the DCF therapy) of docetaxel, cisplatin and 5-fluorouracil to esophageal cancer patients and the severity of febrile neutropenia or the severity of diarrhea (Table 2) .

In addition, in the patients who did not develop febrile neutropenia during the DCF therapy administration, the number of bacteria of *A*. *hadrus* was significantly higher before and after the treatment than in the patients who developed febrile neutropenia (Fig. 1). Even in the analysis of only a group receiving synbiotics therapy in addition to the DCF therapy, in the patients who did not develop febrile neutropenia during the administration of the therapy, the number of bacteria of *A*. *hadrus* tended to be higher before and after the treatment than in the patients who developed febrile neutropenia (Fig. 2). Here, it has been reported that the synbiotics therapy markedly reduces febrile neutropenia and severe diarrhea caused by the DCF therapy (Non Patent Literature 1), and *Lacticaseibacillus paracasei* strain Shirota (LcS) and *Bifidobacterium breve* strain Yakult (BbrY) (*Bifidobacterium breve* YIT 12272 (FERM BP-11320)) are used as probiotics, and galacto-oligosaccharides are used as prebiotics. Incidentally, LcS is a bacterial strain known as *Lactobacillus casei* YIT9029 (FERM BP-1366) before reclassification of *Lactobacillus* bacteria in 2020 (Zheng J et al. Int J Syst Evol Microbiol. 2020 Apr; 70(4): 2782-2858), and has been deposited with National Institute of Advanced Industrial Science and Technology Patent Organism Depositary (currently, the National Institute of Technology and Evaluation Patent Microorganisms Depositary) on January 12, 1981. From the results of logistic analysis, it was suggested that the low number of bacteria and the low occupancy rate of *A*. *hadrus* before the DCF therapy are risk factors for the onset of febrile neutropenia.

Furthermore, in the patients who did not develop diarrhea or had low severity of diarrhea during the DCF therapy administration (an increase in stool frequency was 6 times/day or less as compared with the baseline), the number of bacteria of *A*. *hadrus* was significantly higher before and after the treatment than in the patients who developed extremely severe diarrhea (an increase in stool frequency was 7 times/day or more as compared with the baseline, the presence of fecal incontinence, and requiring hospitalization or emergency treatment) (Fig. 3). Even in the analysis of only the group receiving the synbiotics therapy in addition to the DCF therapy, in the patients who did not develop diarrhea or had low severity of diarrhea during the administration of the therapy, the number of bacteria of *A*. *hadrus* tended to be higher before and after the treatment than in the patients who developed extremely severe diarrhea (Fig. 4). From the results of logistic analysis, it was suggested that the low number of bacteria and the low occupancy rate of *A*. *hadrus* before the DCF therapy are risk factors for exacerbation of diarrhea.

Meanwhile, in the multimodal treatment including not only drug therapy such as the DCF therapy but also a surgical therapy, a radiation treatment, and a combination thereof, digestive tract mucosa, particularly intestinal mucosa, is generally impaired. Furthermore, so-called bacterial translocation in which bacteria in the intestines migrate into a living body occurs together with a damage of immune function of a host. This is known to cause adverse events and infectious complications.

Therefore, *A. hadrus* in the sample can be used as an index for determination of the risk of an adverse event not only during the drug therapy such as the DCF therapy but also during the multimodal treatment.

The multimodal treatment includes a drug therapy, a surgical therapy, a radiation treatment, and a combination thereof, and is preferably a drug therapy, more preferably a drug therapy using an anticancer agent, and further preferably a drug therapy (the DCF therapy) using docetaxel, cisplatin, and 5-fluorouracil. The DCF therapy is known as one of treatments for esophageal cancer.

In addition, the adverse event to be subjected to risk determination is not particularly limited, and examples thereof include blood and lymphatic system disorders, cardiac disorders, ear and labyrinthine disorders, endocrine disorders, eye disorders, gastrointestinal disorders, general and systemic disorders as well as administration site disorders, hepatobiliary disorders, immune system disorders, metabolic and nutritional disorders, musculoskeletal and connective tissue disorders, nervous system disorders, psychiatric disorders, renal and urinary tract disorders, reproductive and breast disorders, respiratory, thoracic and mediastinal disorders, skin and subcutaneous tissue disorders, and vascular disorders. The method of the present invention is suitably used for determining at least one risk selected from the group consisting of blood and lymphatic system disorders and gastrointestinal disorders as adverse events, and specifically, is suitably used for determining at least one risk selected from the group consisting of febrile neutropenia and diarrhea, that is, for determing at least one risk selected from the group consisting of a risk of febrile neutropenia and a risk of diarrhea.

The severity of febrile neutropenia is classified according to CTCAE v4.0 into the following grades.
Grade 1: (not defined)
Grade 2: (not defined)
Grade 3: absolute neutrophil count of < 1,000/mm³ with fever exceeding 38.3°C at least once or fever of 38.0°C or higher sustained more than 1 hour.
Grade 4: life threatening; emergency treatment is required.
Grade 5: Death

In addition, the severity of diarrhea is classified into the following grades according to CTCAE v4.0. Incidentally, the baseline refers to bowel habit (stool frequency) in daily life.

Grade 1: an increase in stool frequency of < 4 times/day as compared with the baseline; a mild increase in amount of colostomy output as compared with the baseline.

Grade 2: an increase in stool frequency of 4 to 6 times/day as compared with the baseline; a moderate increase in amount of colostomy output as compared with the baseline.

Grade 3: an increase in stool frequency of 7 times/day or more as compared with the baseline; fecal incontinence; hospitalization is required; severe increase in amount of colostomy output as compared with the baseline; limitation of self care activities of daily living.

Grade 4: life threatening; emergency treatment is required.

Grade 5: Death

In the method of the present invention, the risk of an adverse event during the multimodal treatment may be determined by using the number of bacteria of *A*. *hadrus* in the sample as an index. Specifically, it can be determined that the risk of an adverse event during the multimodal treatment is higher as the number of bacteria of *A*. *hadrus* in the sample is smaller, whereas the risk of an adverse event during the multimodal treatment is lower as the number of bacteria of *A*. *hadrus* in the sample is larger. Such risk determination is preferably performed by comparing the number of bacteria of *A*. *hadrus* in the sample with a reference value (a cut-off value) set in advance according to the level of the risk. The reference value can be set, for example, by analyzing, using a statistical analysis method, a result of follow-up study on the presence or absence of the onset and/or the degree of exacerbation of an adverse event during the multimodal treatment for a plurality of subjects for whom the number of bacteria of *A*. *hadrus* has been confirmed in advance. Examples of statistical analysis methods include, for example, receiver operating characteristic (ROC) analysis, and for example, a value capable of identifying a high-risk adverse event group can be used as the reference value. In a case where the number of bacteria of *A*. *hadrus* in the sample is equal to or less than the reference value, it can be determined that the risk of an adverse event during the multimodal treatment is high. Meanwhile, in a case where the number of bacteria of *A*. *hadrus* in the sample is larger than the reference value, it can be determined that the risk of an adverse event during the multimodal treatment is low.

In the method of the present invention, the risk of onset of an adverse event during the multimodal treatment may be determined by using the number of bacteria of A. *hadrus* in the sample as an index. Specifically, it can be determined that the risk of onset of an adverse event during the multimodal treatment is higher as the number of bacteria of *A*. *hadrus* in the sample is smaller, whereas the risk of onset of an adverse event during the multimodal treatment is lower as the number of bacteria of *A*. *hadrus* in the sample is larger. Such risk determination is preferably performed by comparing the number of bacteria of *A. hadrus* in the sample with the reference value set in advance according to the level of the risk. The reference value can be appropriately set by those skilled in the art as described above. In a case where the number of bacteria of *A*. *hadrus* in the sample is equal to or less than the reference value, it can be determined that the risk of onset of an adverse event during the multimodal treatment is high. Meanwhile, in a case where the number of bacteria of *A*. *hadrus* in the sample is larger than the reference value, it can be determined that the risk of onset of an adverse event during the multimodal treatment is low. In one example, when the adverse event is febrile neutropenia, the risk of onset of febrile neutropenia during the multimodal treatment can be determined to be high in a case where the number of bacteria of *A*. *hadrus* in the sample is 10^{7.7} or less per gram of the sample, whereas the risk of onset of febrile neutropenia during the multimodal treatment can be determined to be low in a case where the number of bacteria of *A*. *hadrus* in the sample is more than 10^{7.7} per gram of the sample. In another example, when the adverse event is diarrhea, it can be determined that the risk of onset of diarrhea during the multimodal treatment is high in a case where the number of bacteria of *A*. *hadrus* in the sample is 10^{7.6} or less per gram of the sample, whereas it can be determined that the risk of onset of diarrhea during the multimodal treatment is low in a case where the number of bacteria of *A*. *hadrus* in the sample is more than 10^{7.6} per gram of the sample. The determination of the risk of onset of an adverse event during the multimodal treatment is particularly suitably applicable in a case where the adverse event is febrile neutropenia.

In addition, in the method of the present invention, the risk of exacerbation of an adverse event during the multimodal treatment may be determined by using the number of bacteria of *A*. *hadrus* in the sample as an index. Specifically, it can be determined that the smaller the number of bacteria of *A*. *hadrus* in the sample, the higher the risk of exacerbation of an adverse event during the multimodal treatment, whereas the larger the number of bacteria of *A*. *hadrus* in the sample, the lower the risk of exacerbation of an adverse event during the multimodal treatment. Such risk determination is preferably performed by comparing the number of bacteria of *A*. *hadrus* in the sample with the reference value set in advance according to the level of the risk. The reference value can be appropriately set by those skilled in the art as described above. In a case where the number of bacteria of *A*. *hadrus* in the sample is equal to or less than the reference value, it can be determined that the risk of exacerbation of an adverse event during the multimodal treatment is high. Meanwhile, in a case where the number of bacteria of *A*. *hadrus* in the sample is larger than the reference value, it can be determined that the risk of exacerbation of an adverse event during the multimodal treatment is low. In one example, when the adverse event is febrile neutropenia, it can be determined that the risk of exacerbation of febrile neutropenia during the multimodal treatment is high in a case where the number of bacteria of *A*. *hadrus* in the sample is 10^{7.7} or less per gram of the sample, whereas it can be determined that the risk of exacerbation of febrile neutropenia during the multimodal treatment is low in a case where the number of bacteria of *A*. *hadrus* in the sample is more than 10^{7.7} per gram of the sample. More specifically, it can be determined that there is a high possibility that the febrile neutropenia during the multimodal treatment corresponds to the above grade 3 in a case where the number of bacteria of *A*. *hadrus* in the sample is 10^{7.7} or less per gram of the sample, whereas it can be determined that there is a high possibility that the febrile neutropenia during the multimodal treatment corresponds to the above grade 0 in a case where the number of bacteria of *A*. *hadrus* in the sample is more than 10^{7.7} per gram of the sample. In another example, when the adverse event is diarrhea, it can be determined that the risk of exacerbation of diarrhea during the multimodal treatment is high in a case where the number of bacteria of *A. hadrus* in the sample is 10^{7.6} or less per gram of sample, whereas it can be determined that the risk of exacerbation of diarrhea during the multimodal treatment is low in a case where the number of bacteria of *A*. *hadrus* in the sample is more than 10^{7.6} per gram of sample. More specifically, it can be determined that there is a high possibility that diarrhea during the multimodal treatment corresponds to the above grade 3 or higher, for example, grade 3 to 4 in a case where the number of bacteria of *A*. *hadrus* in the sample is 10^{7.6} or less per gram of the sample, whereas it can be determined that there is a high possibility that diarrhea during the multimodal treatment corresponds to the above grade 0 to 2 in a case where the number of bacteria of *A*. *hadrus* in the sample is more than 10^{7.6} per gram of the sample. The determination of the risk of exacerbation of an adverse event is particularly suitably applicable when the adverse event is diarrhea.

Alternatively, in the method of the present invention, the risk of an adverse event during the multimodal treatment may be determined by using the occupancy rate of *A. hadrus* in total bacteria in the sample as an index. Specifically, it can be determined that the risk of an adverse event during the multimodal treatment is higher as the occupancy rate of *A*. *hadrus* in total bacteria in the sample is lower, whereas the risk of an adverse event during the multimodal treatment is lower as the occupancy rate of *A*. *hadrus* in total bacteria in the sample is higher. Such risk determination is preferably performed by comparing the occupancy rate of *A*. *hadrus* in total bacteria in the sample with a reference value set in advance according to the level of the risk. The reference value can be set, for example, by analyzing, using a statistical analysis method, a result of follow-up study on the presence or absence of the onset and/or the degree of exacerbation of an adverse event during the multimodal treatment for a plurality of subjects for whom the occupancy rate of *A*. *hadrus* in total bacteria has been confirmed in advance. Examples of statistical analysis methods include, for example, receiver operating characteristic (ROC) analysis, and for example, a value capable of identifying a high-risk adverse event group can be used as the reference value. In a case where the occupancy rate of *A*. *hadrus* in total bacteria in the sample is equal to or less than the reference value, it can be determined that the risk of an adverse event during the multimodal treatment is high. Meanwhile, when the occupancy rate of *A*. *hadrus* in total bacteria in the sample is higher than the reference value, it can be determined that the risk of an adverse event during the multimodal treatment is low.

In the method of the present invention, the risk of onset of an adverse event during the multimodal treatment may be determined by using the occupancy rate of *A*. *hadrus* in total bacteria in the sample as an index. Specifically, it can be determined that the risk of developing an adverse event during the multimodal treatment is higher as the occupancy rate of *A*. *hadrus* in total bacteria in the sample is lower, whereas the risk of developing an adverse event during the multimodal treatment is lower as the occupancy rate of *A*. *hadrus* in total bacteria in the sample is higher. Such risk determination is preferably performed by comparing the occupancy rate of *A*. *hadrus* in total bacteria in the sample with a reference value set in advance according to the level of the risk. The reference value can be appropriately set by those skilled in the art as described above. When the occupancy rate of *A*. *hadrus* in total bacteria in the sample is equal to or less than the reference value, it can be determined that the risk of onset of an adverse event during the multimodal treatment is high. Meanwhile, when the occupancy rate of *A*. *hadrus* in total bacteria in the sample is higher than the reference value, it can be determined that the risk of onset of an adverse event during the multimodal treatment is low. In one example, when the adverse event is febrile neutropenia, it can be determined that the risk of onset of febrile neutropenia during the multimodal treatment is high in a case where the occupancy rate of *A*. *hadrus* in total bacteria in the sample is 0.25% or less, whereas it can be determined that the risk of onset of febrile neutropenia during the multimodal treatment is low in a case where the occupancy rate of *A*. *hadrus* in total bacteria in the sample is higher than 0.25%. In another example, when the adverse event is diarrhea, it can be determined that the risk of onset of diarrhea during the multimodal treatment is high in a case where the occupancy rate of *A*. *hadrus* in total bacteria in the sample is 0.084% or less, whereas it can be determined that the risk of onset of diarrhea during the multimodal treatment is low in a case where the occupancy rate of *A*. *hadrus* in total bacteria in the sample is higher than 0.084%. The determination of the risk of onset of an adverse event during the multimodal treatment is particularly suitably applicable in a case where the adverse event is febrile neutropenia.

In addition, in the method of the present invention, the risk of exacerbation of an adverse event during the multimodal treatment may be determined by using the occupancy rate of *A*. *hadrus* in total bacteria in the sample as an index. Specifically, it can be determined that the risk of exacerbation of an adverse event during the multimodal treatment is higher as the occupancy rate of *A*. *hadrus* in total bacteria in the sample is lower, whereas the risk of exacerbation of an adverse event during the multimodal treatment is lower as the occupancy rate of *A*. *hadrus* in total bacteria in the sample is higher. Such risk determination is preferably performed by comparing the occupancy rate of *A*. *hadrus* in total bacteria in the sample with a reference value set in advance according to the level of the risk. The reference value can be appropriately set by those skilled in the art as described above. In a case where the occupancy rate of *A*. *hadrus* in total bacteria in the sample is equal to or less than the reference value, it can be determined that the risk of exacerbation of an adverse event during the multimodal treatment is high. Meanwhile, when the occupancy rate of *A. hadrus* in total bacteria in the sample is higher than the reference value, it can be determined that the risk of exacerbation of an adverse event during the multimodal treatment is low. In one example, when the adverse event is febrile neutropenia, it can be determined that the risk of exacerbation of febrile neutropenia during the multimodal treatment is high in a case where the occupancy rate of *A*. *hadrus* in total bacteria in the sample is 0.25% or less, whereas it can be determined that the risk of exacerbation of febrile neutropenia during the multimodal treatment is low in a case where the occupancy rate of *A*. *hadrus* in total bacteria in the sample is higher than 0.25%. More specifically, it can be determined that there is a high possibility that the febrile neutropenia during the multimodal treatment corresponds to the above grade 3 in a case where the occupancy rate of *A*. *hadrus* in total bacteria in the sample is 0.25% or less, whereas it can be determined that there is a high possibility that the febrile neutropenia during the multimodal treatment corresponds to the above grade 0 in a case where the occupancy rate of *A*. *hadrus* in total bacteria in the sample is higher than 0.25%. In another example, when the adverse event is diarrhea, it can be determined that the risk of exacerbation of diarrhea during the multimodal treatment is high in a case where the occupancy rate of *A*. *hadrus* in total bacteria in the sample is 0.084% or less, whereas it can be determined that the risk of exacerbation of diarrhea during the multimodal treatment is low in a case where the occupancy rate of *A*. *hadrus* in total bacteria in the sample is higher than 0.084%. More specifically, it can be determined that there is a high possibility that the diarrhea during the multimodal treatment corresponds to the above grade 3 or higher, for example, grade 3 to 4 in a case where the occupancy rate of *A*. *hadrus* in total bacteria in the sample is 0.084% or less, whereas it can be determined that there is a high possibility that the diarrhea during the multimodal treatment corresponds to the above grade 0 to 2 in a case where the occupancy rate of *A*. *hadrus* in total bacteria in the sample is higher than 0.084%. The determination of the risk of exacerbation of an adverse event is particularly suitably applicable when the adverse event is diarrhea.

The method of the present invention is preferably carried out before or at the beginning of a multimodal treatment (e.g. during or after completion of one course in a multimodal treatment including a plurality of courses), and more preferably before the multimodal treatment.

Since the risk of an adverse event during the multimodal treatment can be determined at an early stage, particularly before the treatment according to the method of the present invention, it is possible to make a more appropriate treatment plan for an individual patient, which in turn leads to improvement in quality of life and therapeutic effect of the patient.

According to the method of the present invention, a patient determined to have a low risk of an adverse event during the multimodal treatment can be subjected to scheduled multimodal treatment, and a patient determined to have a high risk of an adverse event during the multimodal treatment can be subjected to treatment for preventing or reducing an adverse event, such as reduction in the amount of a drug or combination use of synbiotics.

In order to perform the method of the present invention, it is preferable to use a kit including a protocol for measuring *A*. *hadrus* in a sample. The kit includes a measurement reagent and a protocol (descriptions of the method for measuring *A*. *hadrus,* criteria for determining the risk of an adverse event during the multimodal treatment, and factors affecting the measurement results and the degree of their influence, for example) for *A. hadrus.* The determination can be made as in the method using the criteria. Here, examples of the reagent for measuring *A*. *hadrus* include the above-described reagent for measuring the number of intestinal bacteria, reagent for detecting RNA, reagent for detecting DNA, primer capable of specifically detecting *A*. *hadrus,* and primer capable of detecting intestinal bacteria, for example.

Furthermore, from the above, it is considered that an agent for reducing the risk of an adverse event during the multimodal treatment can be screened by using a variation in the number of bacteria or the occupancy rate of *A*. *hadrus,* preferably the variation in the number of bacteria, as an index. Here, the "variation in the number of bacteria" used as an index is a concept including a case where the number of bacteria of *A*. *hadrus* increases after administration of a test substance, a case where an increase in the number of bacteria of *A*. *hadrus* is promoted in comparison before and after administration, and a case where a decrease in the number of bacteria of *A*. *hadrus* is suppressed in comparison before and after administration. In other words, it is determined that the test substance which, in vitro or in vivo, has increased the number of bacteria of *A*. *hadrus,* has promoted the increase in the number of bacteria of *A*. *hadrus,* and has suppressed the decrease in the number of bacteria of *A*. *hadrus* has a reduction effect on the risk of an adverse event during the multimodal treatment. In addition, a "variation in occupancy rate" used as an index is a concept including a case where the occupancy rate of *A*. *hadrus* increases after administration of the test substance, a case where the increase in the occupancy rate of *A*. *hadrus* is promoted in comparison before and after administration, and a case where a decrease in the occupancy rate of *A*. *hadrus* is suppressed in comparison before and after administration. In other words, it is determined that the test substance which, in vitro or in vivo, has increased occupancy rate of *A. hadrus,* has promoted the increase in occupancy rate, and has suppressed the decrease in occupancy rate has a reduction effect on the risk of an adverse event during the multimodal treatment.

For example, by administrating the test substance to a human or a laboratory animal such as a mouse, a rat or a rabbit and comparing with an unadministered human or laboratory animal, it is determined that whether or not the test substance makes the number of bacteria or the occupancy rate of *A*. *hadrus* in the sample vary. When the test substance is determined to be a substance which has increased the number of bacteria of *A*. *hadrus,* has promoted the increase in the number of bacteria of *A*. *hadrus,* and has suppressed the decrease in the number of bacteria of *A*. *hadrus,* the test substance can be used as an agent for reducing the risk of an adverse event during the multimodal treatment. Alternatively, when it is determined that the test substance is a substance which has increased the occupancy rate of *A*. *hadrus,* has promoted the increase in the occupancy rate of *A*. *hadrus,* or has suppressed the decrease in the occupancy rate of *A*. *hadrus,* the test substance can be used as an agent for reducing the risk of an adverse event during the multimodal treatment.

### Examples

Hereinafter, the present invention will be described in more detail with reference to the examples, but the present invention is not limited to the following examples.

### Example 1

### 1. Methods

### (1) Analysis target

81 patients with esophageal cancer aged 20 to 80 years, who were scheduled for preoperative chemotherapy, were enrolled in the study. Among them, 73 subjects (38 subjects in a prophylactic antibiotic administration group and 35 subjects in a synbiotics administration group) whose feces were collected at the time of enrollment and on the 8th day after the start of DCF therapy were analyzed. All patients participating in the study agreed to participate in the study by written consent prior to participation.

### (2) Treatment

In the DCF therapy, docetaxel of 70 mg/m² and cisplatin of 70 mg/m² were administered by infusion on Day 1, and 5-fluorouracil of 700 mg/m² was administered by continuous infusion from Day 1 to Day 5. Basically, chemotherapy was performed for 2 courses at an interval of 3 weeks. The patients in the prophylactic antibiotic administration group took levofloxacin of 500 mg once a day from Day 5 to Day 15 of each course of chemotherapy. In addition to a RACOL-NF liquid for enteral use (Otsuka Pharmaceutical Co., Ltd.) of 600 mL per day from 3 days before the start to Day 12 of each course of chemotherapy, the patients in the synbiotics administration group orally took Yakult BL *Seichoyaku* (intestinal medicine) (Yakult Honsha Co., Ltd.) of 3 g per day, containing 3 × 10⁸ cells or more of viable bacteria of each of *Lacticaseibacillus paracasei* strain Shirota (*Lacticaseibacillus paracasei* YIT 9029: LcS) (*Lactobacillus casei* YIT 9029 (FERM BP-1366) before reclassification of *Lactobacillus* bacteria in 2020) and *Bifidobacterium breve* strain Yakult (BbrY) (*Bifidobacterium breve* YIT 12272 (FERM BP-11320)) as probiotics, and Oligomate S-HP (Yakult Pharmaceutical Industry Co., Ltd.) of 15 mL per day (containing 5 g of galacto-oligosaccharide) as prebiotics from 3 days before the start of one course of chemotherapy until the end of chemotherapy.

### (3) Evaluation of chemotherapy-derived toxicity

Toxicity of febrile neutropenia and diarrhea, which are adverse events of chemotherapy, was evaluated using the criteria of the Common Terminology Criteria for Adverse Events v4.0 (CTCAE v4.0) published by the National Cancer Institute (NCI).

### (4) Collection of fecal sample

At the time of test enrollment and on Day 8 of the first course of chemotherapy, approximately 1.0 g of fresh feces were collected with a collection spoon into preweighed tubes containing 2 mL of RNAlater (Ambion) and ϕ 5 mm zirconia beads. After being shaken and suspended, it was stored at 4°C until a subsequent operation. A primary treatment of the fecal sample used for nucleic acid extraction was performed by the following method. The weight of the fecal sample was measured, and then RNALater was added so as to be 10 times the volume. This was shaken using ShakeMaster Auto (Biomedical science) at 1,048 rpm for 10 minutes. For RNA extraction, 40 µL of a fecal suspension was transferred to a new 2 mL tube to which 1 mL of PBS was added, centrifuged (4°C, 13,000 × g, 5 minutes), and then the supernatant was removed by decantation. This pellet was stored at -80°C until RNA extraction. For DNA extraction, 200 µL of the suspension was transferred to a new 2 mL tube to which 1 mL of PBS added and agitated by a vortex. This was centrifuged (4°C, 13,000 × g, 5 minutes), and then 1 mL of the supernatant was removed. 1 mL of PBS was added again, and the mixture was suspended and centrifuged (4°C, 13,000 × g, 5 minutes), and then 1 mL of the supernatant was removed. The obtained 200 µL suspension was stored at -30°C until DNA extraction.

### (5) 16S rRNA gene amplicon analysis by next-generation sequencer

Extraction of DNA from fecal samples was performed according to the manual using a QIAamp DNA Stool Mini Kit (QIAGEN GmbH). The 16S rRNA gene amplicon analysis was performed as follows. A V1-V2 region of the 16S rRNA gene of each sample was amplified by ABI 7500 Real-Time PCR System (Thermo Fisher Scientific) using a 27Fmod2 forward primer and a 338R reverse primer (SEQ ID NOs: 1 and 2). As a PCR reaction solution (50 µL), 2 × SYBR Premix Ex Tag II (50 µL, Takara Bio), Nuclease-Free Water (22 µL), each primer (100 nM, 1 µL), and template DNA (10 ng/mL, 1 µL) were mixed. The PCR reaction was performed under the condition of initial denaturation by heating at 95°C for 30 seconds, followed by 25 cycles of 95°C for 5 seconds, 55°C for 30 seconds, and 72°C for 40 seconds. An amplification product was purified with AMPure XP Kit (Beckman Coulter Genomics) and quantified with Quant-iT PicoGreen dsDNA Kit (Invitrogen). The amplification products in each sample were mixed so as to be equal amounts to prepare a library, and sequencing was performed on the MiSeq platform (Illumina) using the MiSeq Reagent Kits v2 (Illumina). As a result, 2,621,476 amplicon sequence reads (9,158 to 36,003 reads per sample) were obtained.

### (6) Processing of 16S rRNA gene sequence data

QIIME2 (ver.2019.10, https://qiime2.org/) was used to process the sequence data. A quality control of sequence data was performed using a DADA2 plug-in, and then the sequence data was summarized into ASV (amplicon sequence variant, and the occupancy rate of each ASV for each subject was calculated. For assignment of the phylogenetic information, SILVA 138 database (https://www.arb-silva.de/) was used as a database.

### (7) Design and validation of Anaerostipes hadrus species-specific primers

Multiple alignments of *A*. *hadrus* and related species were constructed by ClustalX using the 16S rRNA sequence obtained from the DDBJ/GenBank/EMBL database. Based on this alignment, a primer set including an AH7F forward primer and an AH1R reverse primer (SEQ ID NOs: 3 and 4) which were specific to the 16S rRNA gene of *A*. *hadrus* was designed using Primer3plus software (http://www.bioinformatics.nl/cgi-bin/primer3plus/primer3plus.cgi). Quantitative RT-PCR was performed using the RNA extracted from cultured *A*. *hadrus* as a template by QuantStudio 12K Flex Real-Time PCR System (Thermo Fisher) using the primer set. Qiagen OneStep RT-PCR kit (Qiagen) was used for quantitative RT-PCR. As a PCR reaction solution (10 µL), 1 × Qiagen Onestep RT-PCR Buffer (2 µL), 0.5 × Q-solution (2 µL), dNTP Mixture (400 µM each), 100,000 fold diluted SYBR Green I (Molecular Probes), Qiagen OneStep RT-PCR enzyme mix (0.4 µL), AH7F forward primer and AH1R reverse primer (0.6 µM), and template RNA (5 µL) were mixed. The RT-PCR reaction was performed under the condition of reverse transcription reaction by heating at 50°C for 30 minutes, initial denaturation by heating at 95°C for 15 minutes, followed by 40 cycles of 94°C for 5 seconds, 55°C for 30 seconds, and 72°C for 50 seconds, and as a result, it was confirmed that the amplification product was obtained when using RNA extracted from YIT10092^{T} (DSM3319^{T}), which is a reference strain of *A*. *hadrus.* In addition, using Primer-Blast program (https://www.ncbi.nlm.nih.gov/tools/primer-blast/), it was confirmed that a non-specific amplification product by a designed primer was not present in an existing database. Furthermore, specificity of the designed primer was verified by performing quantitative RT-PCR under the above conditions using the RNA (equivalent to 10⁵ cells) extracted from 23 species of main constituent bacteria of the intestinal bacterial flora which is a standard strain of Yakult Intestinal Flora-SCAN (YIF-SCAN (registered trademark)), and as a result, it was confirmed that the primer did not show cross-reactivity to any bacterial species.

### (8) Quantification of the number of bacteria of A. hadrus by RT-qPCR

Extraction of total RNA from the fecal sample was performed by improving the AGPC (acid guanidinium thiocyanate-phenol-chloroform extraction) method. Thawed samples were resuspended in a mixed solution of 346.5 µL RLT Buffer (Qiagen), 3.5 µL β-mercaptoethanol (Sigma-Aldrich), and 100 µL Tris-EDTA buffer. Glass beads (0.1 mm, 300 mg, BioSpec Products) were added and shaken using ShakeMaster Auto (Biomedical science) at 1,048 rpm for 5 minutes. Water-saturated phenol (500 µL) was added, and the mixture was incubated at 60°C for 10 minutes. Then, chloroform-isoamyl alcohol (24 : 1, 100 µL) was added, and the mixture was stirred and centrifuged (4°C, 14,000 × g, 5 minutes). 470 µL of the supernatant was collected, and the same amount of chloroform-isoamyl alchol (24 : 1) was added and stirred. 400 µL of the supernatant was collected, and RNA was precipitated and recovered by isopropanol precipitation, and then dissolved in 200 µL of nuclease-free water (Ambion). YIF-SCAN (registered trademark) based on a quantitative RT-PCR method (RT-qPCR) as a basic principle was used for quantification of *A*. *hadrus* in the samples. For RT-qPCR, bacterial species specific primers targeting 16S rRNA against *A*. *hadrus* (SEQ ID NOs: 3 and 4) and reaction conditions described above were used. A threshold cycle (Cq) value in a linear range of the assay was substituted into a standard curve to determine a corresponding bacterial count in each sample. From this, the number of bacteria in each sample was determined. The standard curve was generated using the Cq values and a dilution series of the standard strain *A*. *hadrus* YIT 10092^{T} (DSM3319^{T}) whose cell count has been determined by DAPI staining. A limit of detection (LOD) was defined as the smallest number of bacteria in the standard curve (10⁻² cells/reaction) .

The sequences of the primers used in the above (5), (7) and (8) are shown in the following Table 1.

**[Table 1]**

| Primer | Sequence (5' to 3') | SEQ ID NOs. |
|---|---|---|
| 27Fmod2 | AATGATACGGCGACCACCGAGATCTACACTCTTTCCCTACACGACG CTCTTCCGATCTAGRGTTYGATYMTGGCTCAG | 1 |
| 338R | CAAGCAGAAGACGGCATACGAGATNNNNNNNNNNNNGTGACTGGAG TTCAGACGTGTGCTCTTCCGATCTGCTGCCWCCCGTAGGWGT | 2 |
| AH7F | CGGAACTGAAGATTTGGTGAT | 3 |
| AH1R | GTACCACCGGAGTTTTTACCC | 4 |

| | | |
|---|---|---|
| * In the table, an underlined part indicates a sequence that hybridizes with 16S rRNA gene. | | |

### (9) Sequencing of RT-qPCR amplification products

In order to confirm whether the amplification product was derived from a target microorganism, the bacterial species amplified by RT-qPCR was estimated by sequence analysis. The RT-qPCR product (10 µL) was purified by MultiScreen (registered trademark) Filter Plate (Merck Millipore). Cycle sequence reactions were performed using a BigDye (registered trademark) Terminator version 3.1 Cycle Sequencing kit (Applied Biosystems) according to a manufacturer's manual. The amplification products were purified by ethanol precipitation, dissolved in formamide (1 µL) for denaturation, and sequenced on an ABI PRISM 3130 Genetic Analyzer (Applied Biosystems). Comparison of the obtained rRNA sequences to make an assignment to a particular species was done using the NCBI BLAST program (http://blast.ncbi.nlm.nih.gov/Blast.cgi) .

### (10) Statistical analysis

R (ver 3.6.0) (https://www.r-project.org/) was used for statistical analysis. Continuous variables were represented by median values (a quartile range). A non-parametric Mann-Whitney U test was used to test a difference between the two groups. The difference in the composition of the ASV of the intestinal bacterial flora between the groups was evaluated by ANOVA-Like Differential Expression Analysis (ALDEx) analysis by centered log ratio transformed data. An aldex function (ALDEx2 package) was used for an ALDEx analysis. Regarding the RT-qPCR analysis result, a half value of a lower limit of quantitation was substituted for a sample that was equal to or less than the lower limit of quantitation and used for analysis. In any analysis, p < 0.05 was determined to be a significant difference.

### 2. Results

### (1) Comparison of intestinal bacterial flora after DCF therapy by onset and severity of adverse events

The composition of ASV (amplicon sequence variant) detected on the 8th day after the start of DCF therapy in the prophylactic antibiotic administration group (Antibiotics group) and the synbiotics administration group (Synbiotics group) was compared by the severity of febrile neutropenia, which is an adverse event of the DCF therapy. Patients who did not develop febrile neutropenia during one course of DCF therapy (grade 0) had a significantly higher occupancy rate of ASV15 compared to patients who developed febrile neutropenia (grade 3). As a result of assignment using SILVA as a reference database, ASV15 was estimated to be *Anaerostipes hadrus.*

As a result of analyzing a correlation between the occupancy rate of ASV15 suggested to be associated with adverse events and the severity of febrile neutropenia and diarrhea and obtaining the Spearman's correlation coefficient, ASV15 was found to have a significant negative correlation with the severity of febrile neutropenia and diarrhea (Table 2).

**[Table 2]**

| ASV | Febrile neutropenia | | Diarrhea | |
|---|---|---|---|---|
| | ρ | p | ρ | p |
| ASV 15 (*A. hadrus*) | -0.31 | 0.007 | -0.31 | 0.008 |

### (2) Change in the number of bacteria of A. hadrus by DCF therapy

There was no significant difference between the Antibiotics group and the Synbiotics group in the number of bacteria of *A*. *hadrus* in feces before the DCF therapy. On the 8th day after the start of DCF therapy, the number of bacteria of *A*. *hadrus* tended to decrease (p = 0.11) in the Antibiotics group as compared with that before the DCF therapy, but the number of bacteria of *A*. *hadrus* after the DCF therapy did not decrease in the Synbiotics group. The number of bacteria of *A*. *hadrus* was reduced by the DCF therapy and administration of the prophylactic antibiotic, but such reduction in the number of bacteria was suppressed by internal use of synbiotics.

### (3) Comparison of the number of bacteria of A. hadrus depending on the presence or absence of onset of febrile neutropenia

In the Antibiotics group and the Synbiotics group, the number of bacteria of *A*. *hadrus* was significantly higher before and after the DCF therapy in the patients who did not develop febrile neutropenia during one course of the DCF therapy (grade 0) than in the patients who developed febrile neutropenia (grade 3) (Fig. 1). Also in the analysis of only the Synbiotics group, the number of bacteria of *A*. *hadrus* tended to be higher before and after the DCF therapy in the patients who did not develop febrile neutropenia (grade 0) than in the patients who developed febrile neutropenia (grade 3) (Fig. 2). Therefore, it was suggested that the febrile neutropenia was reduced in patients with a high bacterial count of *A*. *hadrus* in the intestine before the treatment.

### (4) Comparison of the number of bacteria of A. hadrus by severity of diarrhea

In the Antibiotics group and the Synbiotics group, when comparing patients who developed extremely severe diarrhea during one course of the DCF therapy (Grades 3 to 4 : an increase in stool frequency of 7 times/day or more as compared with the baseline, the presence of fecal incontinence, and requiring hospitalization or emergency treatment) with patients who had low severity of diarrhea (grades 0 to 2 : no onset or increase in stool frequency < 6 times/day compared with the baseline), the number of bacteria of *A*. *hadrus* showed a significantly high value in patients with low severity before and after the DCF therapy (Fig. 3). Even in the analysis of only the Synbiotics group, the number of bacteria of *A*. *hadrus* tended to be higher before and after the DCF therapy in the patients with low severity of diarrhea (grades 0 to 2) as compared with the patients who developed extremely severe diarrhea (grades 3 to 4) (Fig. 4). Therefore, it was suggested that diarrhea was reduced in a patient with a high bacterial count of *A*. *hadrus* in the intestine before the treatment.

### (5) Importance of the number of bacteria of A. hadrus in the intestine before DCF therapy in inhibitory effect of synbiotics on febrile neutropenia after DCF therapy

When baseline characteristics were compared according to the presence or absence of the onset of febrile neutropenia in the Synbiotics group, there was a tendency that there was a slight difference in the sex of the patient, but there was no significant difference in the age, BMI, dysphagia score, tumor position, stage, white blood cell count, neutrophil count, total lymphocyte count, and serum albumin of the patient. Thereupon, in order to examine the risk factors of adverse events in the DCF therapy under the synbiotics therapy, a logistic regression was performed using sex of the patient and the number of bacteria of *A*. *hadrus.* The results are shown in Table 3. In Table 3, CI represents a confidence interval, OR represents an odds ratio, and the cut-off value of the number of bacteria of *A*. *hadrus* is a value calculated by receiver operating characteristic (ROC) analysis. In a univariate analysis, the number of bacteria of *A*. *hadrus* before the DCF therapy administration was significantly associated with a decrease in the risk of onset of febrile neutropenia (OR, 0.13; 95% CI, 0.02 to 0.66, p = 0.023). Furthermore, also in a multivariate analysis including the sex of the patient, the number of bacteria of *A*. *hadrus* before the administration of DCF therapy was significantly associated with a decrease in the risk of onset of febrile neutropenia (OR, 0.11; 95% CI, 0.01 to 0.60, p = 0.019).

In addition, the cut-off value was 10^{7.7} per gram of the sample.

**[Table 3]**

| Evaluation items | Factors | Cut-off | Univariate analysis | | Multivariate analysis | |
|---|---|---|---|---|---|---|
| | | | OR (95% CI) | *ρ* value | OR (95% CI) | *ρ* value |
| Febrile neutropenia | Sex (Females vs Males) | - | 6.00 (0.91 to 119.60) | 0.11 | 7.75 (1.00 to 167.81) | 0.088 |
| (Grades 0 vs 3) | *A. hadrus* (Few vs Many) | 7.7 (log₁₀cells/ g of feces) | 0.13 (0.02 to 0.66) | 0.023 | 0.11 (0.01 to 0.60) | 0.019 |

### (6) Importance of occupancy rate of A. hadrus in intestine before DCF therapy in inhibitory effect of synbiotics on febrile neutropenia after DCF therapy

In order to examine the risk factors for adverse events in the DCF therapy under the synbiotics therapy, the logistic regression was performed using the sex of the patient and the occupancy rate of *A*. *hadrus.* The results are shown in Table 4. In Table 4, CI represents a confidence interval, OR represents an odds ratio, and the cut-off value of the occupancy rate of *A*. *hadrus* is a value calculated by receiver operating characteristic (ROC) analysis. In the univariate analysis, the occupancy rate of *A*. *hadrus* before the DCF therapy administration tended to be associated with a reduction in the risk of developing febrile neutropenia (OR, 0.24; 95% CI, 0.05 to 1.02, p = 0.060). Furthermore, also in the multivariate analysis including the sex of the patient, the occupancy rate of *A*. *hadrus* before the DCF therapy administration tended to be associated with the decrease in the risk of onset of febrile neutropenia (OR, 0.20; 95% CI, 0.03 to 0.95, p = 0.051). In addition, the cut-off value was 0.25%.

**[Table 4]**

| Evaluation items | Factors | Cut-off | Univariate analysis | | Multivariate analysis | |
|---|---|---|---|---|---|---|
| | | | OR (95% CI) | *ρ* value | OR (95% CI) | *ρ* value |
| Febrile neutropenia | Sex (Females vs Males) | - | 6.00 (0.91 to 119.60) | 0.11 | 7.36 (0.99 to 157.96) | 0.093 |
| (Grades 0 vs 3) | *A. hadrus* (Few vs Many) | 0.25 (%) | 0.24 (0.05 to 1.02) | 0.060 | 0.20 (0.03 to 0.95) | 0.051 |

### (7) Importance of the number of bacteria of A. hadrus in intestine before DCF therapy in inhibitory effect of synbiotics on diarrhea after DCF therapy

When the baseline characteristics were compared in terms of the degree of severity of diarrhea in the Synbiotics group, no significant difference was observed in age, sex, BMI, dysphagia score, tumor position, stage, white blood cell count, neutrophil count, total lymphocyte count, and serum albumin of the patients. Thereupon, in order to examine the risk factors of adverse events in DCF therapy under the synbiotics therapy, the logistic regression was performed using the number of bacteria of A. *hadrus.* The results are shown in Table 5. In Table 5, CI represents a confidence interval, OR represents an odds ratio, and the cut-off value of the number of bacteria of *A. hadrus* is a value calculated by receiver operating characteristic (ROC) analysis. In the univariate analysis, the number of bacteria of A. *hadrus* before the DCF therapy administration tended to be associated with the decrease in the risk of exacerbation of diarrhea (OR, 0.14; 95% CI, 0.01 to 1.13, p = 0.11). In addition, the cut-off value was 10^{7.6} per gram of the sample.

**[Table 5]**

| Evaluation items | Factors | Cut-off | Univariate analysis | |
|---|---|---|---|---|
| | | | OR (95% CI) | *ρ* value |
| Diarrhea (Grades 0 to 2 vs 3 to 4) | *A. hadrus* (Few vs Many) | 7.6 (log₁₀cells/g of feces) | 0.14 (0.01 to 1.13) | 0.11 |

### (8) Importance of occupancy rate of A. hadrus in intestine before DCF therapy in inhibitory effect of synbiotics on diarrhea after DCF therapy

To examine the risk factors for adverse events in the DCF therapy under the synbiotics therapy, the logistic regression was performed using the occupancy rate of A. *hadrus.* The results are shown in Table 6. In Table 6, CI represents a confidence interval, OR represents an odds ratio, and the cut-off value of the occupancy rate of A. *hadrus* is a value calculated by receiver operating characteristic (ROC) analysis. In the univariate analysis, the occupancy rate of A. *hadrus* before the DCF therapy administration tended to be associated with the decrease in the risk of exacerbation of diarrhea (OR, 0.05; 95% CI, 0.00 to 0.42, p = 0.014). In addition, the cut-off value was 0.084%.

**[Table 6]**

| Evaluation items | Factors | Cut-off | Univariate analysis | |
|---|---|---|---|---|
| | | | OR (950 CI) | *ρ* value |
| Diarrhea (Grades 0 to 2 vs 3 to 4) | *A. hadrus* (Few vs Many) | 0.084 (%) | 0.05 (0.00 to 0.42) | 0.014 |

As described above, *A. hadrus* can be used for determination of the risk of an adverse event during the multimodal treatment, particularly during the drug therapy.

## Claims

1. A method for determining a risk of an adverse event during multimodal treatment, the method comprising measuring *Anaerostipes hadrus* in a sample collected from a subject.

2. The method according to claim 1, wherein the risk of an adverse event is a risk of onset or exacerbation of an adverse event.

3. The method according to claim 1 or 2, wherein the multimodal treatment is a treatment selected from the group consisting of a drug therapy, a surgical therapy, radiation treatment, and a combination thereof.

4. The method according to any one of claims 1 to 3, wherein the multimodal treatment is the drug therapy.

5. The method according to any one of claims 1 to 4, wherein the adverse event is at least one selected from the group consisting of febrile neutropenia and diarrhea.

6. The method according to any one of claims 1 to 5, wherein the sample is a fecal sample of the subject.

7. The method according to any one of claims 1 to 6, further comprising: comparing the number of bacteria of *Anaerostipes hadrus* in the sample with a reference value, and determining that the risk of an adverse event during the multimodal treatment is high when the number of bacteria is equal to or less than the reference value.

8. The method according to any one of claims 1 to 6, further comprising: comparing an occupancy rate of *Anaerostipes hadrus* in total bacteria in the sample with a reference value, and determining that the risk of an adverse event during the multimodal treatment is high when the occupancy rate is equal to or less than the reference value.

9. The method according to any one of claims 1 to 6, further comprising: determining that a risk of febrile neutropenia during the multimodal treatment is high in a case where number of bacteria of *Anaerostipes hadrus* in the sample is 10^{7.7} or less per gram of the sample or an occupancy rate of *Anaerostipes hadrus* in total bacteria in the sample is 0.25% or less.

10. The method according to any one of claims 1 to 6, further comprising: determining that a risk of diarrhea during the multimodal treatment is high when the number of bacteria of *Anaerostipes hadrus* in the sample is 10^{7.6} or less per gram of the sample or an occupancy rate of *Anaerostipes hadrus* in total bacteria in the sample is 0.084% or less.

11. A kit for carrying out the method according to any one of claims 1 to 10, comprising a measurement reagent and a protocol for *Anaerostipes hadrus* in the sample.

12. A method for screening for an agent for reducing the risk of an adverse event during multimodal treatment, the method comprising using the number of bacteria or an occupancy rate of *Anaerostipes hadrus* as an index.
